# EUROPÄISCHE PATENTANMELDUNG

(11) **EP 4 443 908 A1**
(43) Veröffentlichungstag der Anmeldung: **09.10.2024**
(21) Anmeldenummer: 24161055.9
(22) Anmeldetag: 04.03.2024
(51) Int. Cl.: H04R 25/00, A61B 5/01, A61B 5/00, H04R 1/10

(54) **VERFAHREN ZUM BETRIEB EINES HÖRGERÄTESYSTEMS**

(30) Priorität: 28.03.2023 DE 102023202804
(71) Anmelder: Sivantos Pte. Ltd., Singapore 539775 (SG)
(72) Erfinder: KRIEG, Julius, 91058 Erlangen (DE)
(74) Vertreter: FDST Patentanwälte

(57) **Zusammenfassung**

Die Erfindung betrifft ein Verfahren (28) zum Betrieb eines Hörgerätesystems (4), das zwei Hörgeräte (6), insbesondere Hörhilfegeräte, mit jeweils einem Temperatursensor (18) aufweist. Mittels jedes Temperatursensors (18) wird ein aktueller Temperaturwert (32) ermittelt, und eine Abweichung (52) zwischen den beiden Temperaturwerten (32) wird ermittelt. Anhand der Abweichung (52) wird ein Gesundheitszustand (64) eines Nutzers (2) des Hörgerätesystems (4) geschätzt. Ferner betrifft die Erfindung ein Hörgerätesystem (4).

## Beschreibung

Die Erfindung betrifft ein Verfahren zum Betrieb eines Hörgerätesystems und ein Hörgerätesystem. Das Hörgerätesystem weist zwei Hörgeräte mit jeweils einem Temperatursensor auf.

Personen, die unter einer Verminderung des Hörvermögens leiden, verwenden üblicherweise ein Hörhilfegerät. Hierbei wird meist mittels eines Mikrofons, also eines elektromechanischen Schallwandlers, ein Umgebungsschall in ein elektrisches (Audio-/Schall-)Signal gewandelt, sodass ein elektrisches Signal erstellt wird. Die elektrischen Signale werden mittels einer Verstärkerschaltung bearbeitet und mittels eines weiteren elektromechanischen Wandlers in Form eines Hörers in den Gehörgang der Person eingeleitet. Die Verstärkerschaltung ist dabei ein Bestandteil einer Steuereinheit des Hörhilfegeräts. Meist erfolgt zudem eine Bearbeitung der Schallsignale, wofür üblicherweise ein Signalprozessor der Verstärkerschaltung verwendet wird. Hierbei ist die Verstärkung auf einen etwaigen Hörverlust des Hörgeräteträgers abgestimmt.

Meist ist die Verminderung des Hörvermögens altersbedingt. Dabei liegen bei der aufgrund des Alters nutzenden Person oft auch zusätzliche Krankheitsbilder vor, die zu einer Verschlechterung des Gesundheitszustands führen können. Auch ist es möglich, dass aufgrund der Verminderung des Hörvermögens Begleiterkrankungen auftreten, zum Beispiel eine Depression. Deswegen kann eine Antriebslosigkeit vorliegen, die die Person davon abhält, das Hörhilfegerät zu verwenden oder korrekt zu bedienen, sodass die Verminderung des Hörvermögens nicht ausgeglichen wird, was zu einer Verstärkung der Depression führen kann. Meist setzt eine derartige Depression schleichend ein, weswegen diese von der Person selbst und auch von Mitmenschen meist erst vergleichsweise spät erkannt wird. Wenn die Depression erkannt wird, ist diese meist weit fortgeschritten, sodass eine vergleichsweise umfangreiche Behandlung erforderlich ist.

Der Erfindung liegt die Aufgabe zugrunde, ein besonders geeignetes Verfahren zum Betrieb eines Hörgerätesystems ein besonders geeignetes Hörgerätesystem anzugeben, wobei insbesondere ein Komfort und/oder eine Funktionalität erhöht sind.

Hinsichtlich des Verfahrens wird diese Aufgabe durch die Merkmale des Anspruchs 1 und hinsichtlich des Hörgerätesystems durch die Merkmale des Anspruchs 9 erfindungsgemäß gelöst. Vorteilhafte Weiterbildungen und Ausgestaltungen sind Gegenstand der jeweiligen Unteransprüche.

Das Verfahren dient dem Betrieb eines Hörgerätesystems, das zwei Hörgeräte aufweist. Die Hörgeräte sind dabei vorgesehen und eingerichtet, am menschlichen Körper getragen zu werden. Mit anderen Worten werden die Hörgeräte im bestimmungsgemäßen Zustand von einem Träger getragen, der auch als Nutzer oder Benutzer bezeichnet wird. Vorzugsweise umfasst dabei jedes Hörgerät bevorzugt eine Haltevorrichtung, mittels derer eine Befestigung am menschlichen Körper möglich ist. Dabei ist eines der Hörgeräte insbesondere einem linken Ohr des Trägers und das andere einem rechten Ohr des Trägers zugeordnet, sodass es sich um ein binaurales Hörgerätesystem handelt. Beispielsweise sind dabei die beiden Hörgeräte zueinander baugleich. Alternativ hierzu sind diese unterschiedlich, aber zumindest ähnlich. Zweckmäßigerweise sind die beiden Hörgeräte zueinander spiegelbildlich, zumindest äußerlich, ausgestaltet, sodass eine Anpassung an die unterschiedlichen Ohrformen vorhanden ist.

Beispielsweise sind die Hörgeräte Kopfhörer oder umfasst jeweils einen Kopfhörer. Diese sind beispielsweise als sog. In-Ears-, On-Ears- und Over-Ears- Kopfhörer ausgestaltet. Beispielsweise dienen die Kopfhörer der Widergabe von Informationen und/oder Musik, und/oder die Kopfhörer dienen der Störgeräuschunterdrückung und sind sog. Noise Cancelling Kopfhörer. Besonders bevorzugt ist jedes Hörgerät jedoch ein Hörhilfegerät. Die Hörhilfegeräte dient der Unterstützung einer unter einer Verminderung des Hörvermögens leidenden Person. Mit anderen Worten ist jedes Hörhilfegerät ein medizinisches Gerät, mittels dessen beispielsweise ein partieller Hörverlust ausgeglichen wird. Das jeweilige Hörhilfegerät ist beispielsweise ein "Receiver-in-the-canal"-Hörhilfegerät (RIC; Ex-Hörer-Hörhilfegerät), ein Im-Ohr-Hörhilfegerät, wie ein "in-the-ear"-Hörhilfegerät, ein "in-the-canal"-Hörhilfegerät (ITC) oder ein "complete-in-canal"-Hörhilfegerät (CIC). Alternativ ist das jeweilige Hörhilfegerät ein Hinter-dem-Ohr-Hörhilfegerät ("Behind-the-Ear"-Hörhilfegerät), das hinter einer Ohrmuschel getragen wird. Wenn es sich bei dem jeweiligen Hörgerät um ein Hörhilfegerät handelt, ist das jeweilige Hörgerät vorgesehen und eingerichtet, beispielsweise hinter dem zugeordneten Ohr oder innerhalb eines Gehörgangs des jeweiligen Ohrs angeordnet zu werden. Zweckmäßigerweise dabei somit eines der Hörgeräte vorgesehen eingerichtet an oder in dem rechten Ohr und das andere Hörgerät vorgesehen eingerichtet an oder in dem linken Ohr getragen werden.

Jedes Hörgerät umfasst vorzugsweise ein Mikrofon, das dem Erfassen von Schall dient. Insbesondere wird bei Betrieb mittels des jeweiligen Mikrofons ein Umgebungsschall erfasst, oder zumindest ein Teil hiervon. Bei dem jeweiligen Mikrofon handelt es sich insbesondere um einen elektromechanischen Schallwandler. Das jeweilige Mikrofon weist beispielsweise lediglich eine einzige Mikrofoneinheit oder mehrere Mikrofoneinheiten auf, die miteinander wechselwirken. Jede der Mikrofoneinheiten weist zweckmäßigerweise eine Membran auf, die anhand von Schallwellen in Schwingungen versetzt wird, wobei die Schwingungen mittels eines entsprechenden Aufnahmegeräts, wie eines Magneten, der in einer Spule bewegt wird, in ein elektrisches Signal gewandelt wird. Somit ist es möglich, mittels der jeweiligen Mikrofoneinheit ein Audiosignal zu erstellen, das auf dem auf die Mikrofoneinheit auftreffendem Schall basiert. Die Mikrofoneinheiten sind insbesondere unidirektional ausgestaltet. Das Mikrofon ist zweckmäßigerweise zumindest teilweise innerhalb eines Gehäuses des Hörgeräts angeordnet und somit zumindest teilweise geschützt.

Jedes Hörgerät umfasst ferner eine Steuereinheit, die mit dem jeweiligen Mikrofon gekoppelt ist. Die Steuereinheit umfasst zweckmäßigerweise eine Signalverarbeitungseinheit oder ist mittels dieser gebildet. Die Signalverarbeitungseinheit dient dabei insbesondere dem Weiterverarbeiten oder zumindest Analysieren des/der mittels des Mikrofons erstellten Audiosignal(en). Insbesondere erfolgt mittels der Steuereinheit eine Bearbeitung des Audiosignals, sodass ein Ausgabesignal erstellt wird, das im Vergleich zum Audiosignal verändert ist. Insbesondere wird dabei mittels der Steuereinheit eine Verstärkung von bestimmten Frequenzen des Audiosignals durchgeführt, wobei vorzugsweise eine Anpassung an einen etwaigen Hörverlust des Trägers erfolgt. Beispielsweise weist die Signalverarbeitungseinheit mehrere analoge Komponenten auf. Zweckmäßigerweise umfasst die Signalverarbeitungseinheit oder zumindest die Steuereinheit einen digitalen Soundprozessor (DSP). Zweckmäßigerweise ist der (Sound-)Prozessor programmierbar ausgestaltet.

Vorzugsweise weist jedes Hörgerät einen Hörer auf, der insbesondere dem Ausgeben des jeweiligen Ausgabesignals dient. Das jeweilige Ausgabesignal ist hierbei insbesondere ein elektrisches Signal. Zweckmäßigerweise ist der Hörer mit der Steuereinheit gekoppelt, insbesondere signaltechnisch mit dieser verbunden. Je nach Ausgestaltung des Hörgeräts ist im bestimmungsgemäßen Zustand die jeweilige Ausgabevorrichtung zumindest teilweise innerhalb eines Gehörgangs des Trägers des Hörgeräts, also einer Person, angeordnet oder zumindest akustisch mit diesem verbunden.

Insbesondere ist jedes Hörgerät kabellos und dafür vorgesehen und eingerichtet, zumindest teilweise in einen Gehörgang eingeführt zu werden. Besonders bevorzugt umfasst das jeweilige Hörgerät einen Energiespeicher, mittels dessen eine Energieversorgung bereitgestellt ist. Vorzugsweise weist jedes Hörgerät eine Kommunikationseinrichtung auf, die insbesondere ein Funksystem umfasst. Somit ist es möglich, die beiden Hörgeräte signaltechnisch miteinander zu verbinden. Dabei erfolgt beispielsweise bei Betrieb eine Bearbeitung des jeweiligen etwaigen Ausgabesignals in Abhängigkeit des mittels des jeweils anderen Hörgeräts erstellten Audiosignals.

Jedes Hörgerät weist ferner einen Temperatursensor auf. Der Temperatursensor dient dabei dem Erfassen/Messen einer Temperatur und ist hierfür geeignet, insbesondere vorgesehen und eingerichtet. Die den beiden Hörgeräten zugeordneten Temperatursensoren sind zweckmäßigerweise zueinander baugleich, sodass diese in vorzugsweise das gleiche zeitabhängige Verhalten bei Temperaturänderungen aufweisen.

Jeder Temperatursensor dient insbesondere dem Erfassen/Messen einer Temperatur im Bereich des jeweiligen Ohrs des Nutzers oder ist hierfür zumindest geeignet. Zweckmäßigerweise befindet sich im bestimmungsgemäßen Tragezustandes jedes Hörgeräts der jeweilige Temperatursensor im Bereich des Trommelfells des jeweils zugeordneten Ohrs. Der Temperatursensor befindet sich im bestimmungsgemäßen Zustand vorzugsweise im Bereich des jeweiligen Trommelfells und/oder in dem jeweiligen Gehörgang oder ist zumindest dorthin gerichtet. Alternativ hierzu ist der Temperatursensor vorgesehen eingerichtet, eine Temperatur innerhalb eines Gehäuses des jeweiligen Hörgeräts zu erfassen. Beispielsweise wird hierbei der Temperatursensor zudem zum Ermitteln einer Betriebstemperatur des etwaigen Hörgeräts verwendet.

Beispielsweise ist der der jeweilige Temperatursensor ein temperaturabhängiger Widerstand, der im bestimmungsgemäßen (Trage-)Zustand des jeweiligen Hörgeräts im Bereich des jeweiligen Ohrs anliegt, sodass ein mechanischer Kontakt vorhanden ist. Alternativ hierzu handelt es sich bei dem Temperatursensor beispielsweise um einen Infrarotsensor mittels dessen ein kontaktloses Erfassen einer jeweiligen Temperatur ermöglicht ist.

Das Verfahren sieht vor, dass mittels jedes Temperatursensors ein aktueller Temperaturwert ermittelt wird. Insbesondere wird hierfür eine Temperatur mittels des jeweiligen Temperatursensors gemessenen. Beispielsweise wird die Temperatur im Bereich eines des jeweiligen zugeordneten Ohrs/ Gehörgangs gemessen und hieraus der jeweilige aktuelle Temperaturwert erstellt. Beispielsweise ist der aktuelle Temperaturwert, der insbesondere auch lediglich als Temperaturwert bezeichnet wird, ein analoger Wert oder zweckmäßigerweise ein digitaler Wert. Vorzugsweise wird somit für jedes Ohr ein jeweiliger aktueller Temperaturwert ermittelt.

In einem weiteren Arbeitsschritt wird eine Abweichung zwischen den beiden Temperaturwerten ermittelt. Hierfür wird beispielsweise von dem einen Hörgerät der jeweilige Temperaturwert zu dem anderen Hörgerät übertragen. Alternativ hierzu werden die beiden (aktuellen) Temperaturwerte zwischen den beiden Hörgeräten ausgetauscht, und bei jedem der Hörgeräte wird die jeweilige Abweichung ermittelt. Bevorzugt wird die Abweichung vorzeichenbehaftet erstellt. Zum Ermitteln der Abweichung wird insbesondere die Differenz zwischen den beiden Temperaturwerte erstellt und somit der eine von dem anderen abgezogen. Dies erfolgt geeigneterweise stets in der gleichen Art und Weise, sodass zum Beispiel stets von dem linken Ohr zugeordneten Temperaturwert der dem rechten Ohr zugeordnete Temperaturwert abgezogen wird. Alternativ hierzu wird zum Ermitteln der Abweichung lediglich überprüft, welcher der Temperaturwert größer ist, sodass die Abweichung lediglich qualitativ ermittelt wird.

In einem weiteren Arbeitsschritt wird anhand der Abweichung ein Gesundheitszustand des Nutzers des Hörgeräts geschätzt. Zum Schätzen des Gesundheitszustands werden dabei nicht oder zumindest nicht nur der Absolutwert der Temperaturwerte berücksichtigt, sondern die Abweichung, insbesondere die Differenz, zwischen diesen. Somit ist insbesondere ein Kalibrieren der beiden Temperatursensoren nicht erforderlich, weswegen Herstellungskosten reduziert sind.

Bei Vorliegen von manchen Erkrankungen ist die Aktivität des Gehirns des Nutzers weniger gleichmäßig auf beide laterale Seiten (Hemisphären) des Gehirns verteilt als ohne das Vorliegen einer derartigen Erkrankung. Aufgrund der unterschiedlichen Aktivität der Hemisphären ist die Durchblutung weniger symmetrisch, weswegen sich eine Temperaturdifferenz zwischen den beiden Gehirnhälften ergibt. Anhand der Abweichung der Temperaturwerte wird somit ein Rückschluss auf eine unterschiedliche Durchblutung des Gehirns gezogen und hieraus der Gesundheitszustand geschätzt. Bei im Wesentlichen keiner Abweichung wird angenommen, dass keine krankhafte Veränderung vorliegt. Dahingegen wird geschätzt, dass der Gesundheitszustand verschlechtert ist, je größer die Abweichung ist.

Beispielsweise wird zum Schätzen des Gesundheitszustands die Wahrscheinlichkeit für das Vorliegen einer Erkrankung, wie einer Depression, insbesondere einer depressiven Episode, ermittelt. Zum Beispiel erfolgt hierbei lediglich ein binäres Ermitteln für das Vorliegen der Depression, oder es erfolgt zudem auch das Ermitteln der Wahrscheinlichkeit für eine Schwere der Erkrankung, insbesondere der Depression. Mit anderen Worten wird insbesondere eine Wahrscheinlichkeit für das Vorliegen einer leichten Depression, eine Wahrscheinlichkeit für das Vorliegen einer mittleren Depression und/oder eine Wahrscheinlichkeit für das Vorliegen einer schweren Depression ermittelt. Falls insbesondere die Wahrscheinlichkeit für das Vorliegen der Depression erhöht ist, wird insbesondere ein vergleichsweise schlechter Gesundheitszustand geschätzt. Dabei ist die Wahrscheinlichkeit insbesondere erhöht, je größer die Abweichung ist.

Als eine derartige Verschlechterung des Gesundheitszustands wird beispielsweise eine Depression, eine gesteigerte Aggressivität, ein schizoides Verhalten, ein gesellschaftlicher Rückzug und/oder antriebsloser/affektiver Zustand herangezogen. Bei diesen ist die Durchblutung der linken Gehirnhälfte verringert. Zusammenfassend ist insbesondere die Wahrscheinlichkeit für einen verschlechterten Gesundheitszustand je größer, je geringer (kälter) der dem linken Ohr zugeordneten aktuelle Temperaturwert im Vergleich zu der dem rechten Ohr zugeordneten Temperaturwert ist.

Anhand des Verfahrens ist somit ein Gesundheitszustand des Nutzers ableitbar oder zumindest schätzbar, wobei von dem Nutzer keine zusätzliche Tätigkeit durchgeführt werden muss. Somit ist ein Komfort für diesen und eine Funktionalität des Hörgerätesystems erhöht. Hierbei ist es insbesondere möglich, eine mögliche Depression, ein verstärktes aggressives Verhalten, einen schizoiden, unmotivierten, antriebslosen oder affektiven Gesundheitszustand und/oder einen sozialen Rückzug frühzeitig zu erkennen, vorzugsweise das Vorliegen hiervon zu schätzen, sodass nachfolgend von einer medizinischen Fachperson dies gezielt überprüft und eine entsprechende Diagnose erstellt werden kann. Mit anderen Worten wird ein Hinweis für eine medizinische Fachperson oder eine sonstige Person aus dem medizinischen Bereich geben, hinsichtlich des möglichen Vorliegens einer entsprechenden Erkrankung, sodass dies gezielt überprüft werden kann. Somit ist ein vergleichsweise frühzeitiges Erkennen einer derartigen Erkrankung möglich, weswegen bereits frühzeitig eine Behandlung erfolgen kann, bevor die Krankheit vergleichsweise schwer oder unbehandelbar ist. Auch können auf diese Weise die Folgen abgemildert werden, sofern die jeweilige Erkrankung tatsächlich vorliegt.

Beispielsweise wird in Abhängigkeit des geschätzten Gesundheitszustands eine Einstellung eines der Hörgeräte verändert oder zumindest eines Hörgerätesystems. Zum Beispiel wird hierbei eine Meldung ausgegeben, vorzugsweise für den Nutzer oder eine sonstige Person. Beispielsweise wird hierbei die Einstellung nach dem Schätzen des Gesundheitszustands stets verändert oder lediglich dann, wenn eine Änderung des geschätzten Gesundheitszustands vorliegt. Hierfür wird insbesondere nach dem Schätzen des Gesundheitszustands dieser abgespeichert, und das Verfahren wird mehrmals durchgeführt, weswegen der Gesundheitszustand mehrmalig geschätzt und jeweils abgespeichert wird. Somit ist eine Änderung des geschätzten Gesundheitszustands feststellbar, wobei der zeitliche Verlauf des geschätzten Gesundheitszustands vorliegt.

Vorzugsweise werden die beiden Temperaturwerte zwischen den beiden Hörgeräten ausgetauscht, vorzugsweise mittels der etwaigen Kommunikationseinrichtung. Diese basiert beispielsweise auf einem Bluetooth-Standard. Besonders bevorzugt jedoch umfasst das Hörgerätesystem zudem ein tragbares Gerät, wie ein Smartphone, Tablet oder Wearable, auf dem beispielsweise eine sogenannte App oder ein sonstiges Programm abläuft, mittels dessen zumindest teilweise das Verfahren durchgeführt wird. Hierbei werden die Temperaturwerte zweckmäßigerweise zu dem tragbaren Gerät übertragen, mittels dessen insbesondere die Abweichung ermittelt sowie der Gesundheitszustand geschätzt wird. Somit sind Anforderungen an die Hörgeräte verringert, weswegen Herstellungskosten reduziert sind. Beispielsweise ist zwischen dem Ermitteln der beiden Temperaturwerte ein Zeitversatz vorhanden, der beispielsweise der zum Beispiel stochastisch oder stets gleich ist. Besonders bevorzugt jedoch werden die beiden Temperaturwerte zeitgleich ermittelt, sodass insbesondere eine synchrone Messung der jeweiligen Temperaturen erfolgt. Vorzugsweise wird somit bei den Ohren synchron die Temperatur gemessen. Somit wird die Temperatur, die den beiden Ohren zugeordnet ist, im gleichen Zustand des Nutzers ermittelt, weswegen die etwaige Abweichung nicht aufgrund von veränderten Umständen des Nutzers hervorgerufen wird. Geeigneterweise wird somit bei dem Verfahren zunächst eine Synchronisation der beiden Hörgeräte durchgeführt, insbesondere bei einem Neustart des Hörgerätesystems, sodass lediglich einmalig eine entsprechende Synchronisation erforderlich ist. Alternativ hierzu erfolgt eine mehrmalige Synchronisation, beispielsweise jede Stunde, sodass ein sich aufgrund von beispielsweise Fertigungstoleranzen der Hörgeräte ergebender Zeitversatz begrenzt ist. Insbesondere wird beim Messen der jeweiligen Temperatur ein entsprechender Zeitstempel in dem jeweiligen Temperaturwert abgespeichert, der somit nach Art eines Vektors/Tupels ausgebildet ist. Somit ist auch bei einer nachgelagerten Auswertung ermittelbar, zu welchem Zeitpunkt die jeweilige Temperatur ermittelt, insbesondere gemessen, wurde.

Beispielsweise wird lediglich einmalig der jeweilige Temperaturwert ermittelt und somit die Abweichung, anhand derer der Gesundheitszustand geschätzt wird. Mit anderen Worten wird anhand jeder Abweichung jeweils ein entsprechender Gesundheitszustand geschätzt, sodass insbesondere ein vergleichsweise detaillierter Zeitverlauf des geschätzten Gesundheitszustands vorliegt, falls das Verfahren mehrmalig durchgeführt wird. Besonders bevorzugt jedoch wird jeder Temperaturwert mehrmals gemessen, wobei die zeitlichen Abstände dazwischen vorzugsweise konstant sind. Insbesondere ist der zeitliche Abstand dazwischen zwischen 1 Sekunde und 10 Stunden, und vorzugsweise im Wesentlichen gleich einer Stunde oder 30 Minuten. Mit anderen Worten werden jede Stunde zwei entsprechende aktuelle Temperaturwerte ermittelt. Anhand jedes Paars an Temperaturwerten, die insbesondere zeitgleich gemessen werden, wird hierbei jeweils die Abweichung ermittelt. Mit anderen Worten wird in den zeitlichen Abständen für jedes Ohr der entsprechende Temperaturwert ermittelt und hieraus die Abweichung, sodass eine Anzahl an Abweichungen vorliegt. Es wird ein Zentralmaß dieser Abweichungen ermittelt, beispielsweise der Mittelwert, wie der arithmetische Mittelwert oder ein gewichteter Mittelwert. Alternativ hierzu wird als Zentralmaß der Median verwendet.

Der Gesundheitszustand wird dabei anhand des Zentralmaßes geschätzt. Auf diese Weise führt die Abweichung zwischen den beiden Temperaturwerte, die aufgrund von äußeren Umständen hervorgerufen werden, nicht zu einer Verfälschung des geschätzten Gesundheitszustands. Derartige äußere Umstände können dabei sein, dass lediglich eines Ohren einer direkten Sonneneinstrahlung und/oder der direkten Einwirkungen einer Klimaanlage/Heizung ausgesetzt ist. Mit anderen Worten werden aufgrund des Zentralmaßes statistische Fehler nicht oder in geringerem Maße berücksichtigt, sodass eine Genauigkeit beim Schätzen des Gesundheitszustands verbessert ist. Beispielsweise wird zudem eine statistische Eigenschaft des Zentralmaßes bestimmt, wie eine Varianz oder eine Standardabweichung. Insbesondere wird diese beim Schätzen des Gesundheitszustandes berücksichtigt, sofern anhand jedes Zentralmaßes jeweils der Gesundheitszustand geschätzt wird.

Beispielsweise wird der Gesundheitszustand direkt mittels des Zentralmaßes ermittelt. Alternativ hierzu wird für mehrere aufeinanderfolgende Zeitfenster jeweils ein derartiges Zentralmaß erstellt. Der Gesundheitszustand wird dabei anhand eines weiteren Zentralmaßes der Zentralmaße geschätzt wird. Als weiteres Zentralmaß wird beispielsweise der Mittelwert, wie ein arithmetischer oder gewichteter Mittelwert, oder der Median verwendet. Beispielsweise ist die Berechnungsvorschrift zur Ermittlung des weiteren Zentralmaßes gleich der Berechnungsvorschrift zur Ermittlung des Zentralmaßes, oder diese sind unterschiedlich. So wird beispielsweise als weiteres Zentralmaß der Median und als Zentralmaß der Mittelwert verwendet.

Vorzugsweise werden für die Erstellung jedes weiteren Zentralmaßes zwischen 25 und 20 Zentralmaße, zweckmäßigerweise 10 Zentralmaße, verwendet, sodass pro Zeitfenster jeweils 10 Zentralmaße ermittelt werden. Insbesondere sind sämtliche Zeitfenster konstant und/oder zwischen 30 Minuten und 5 Wochen. Vorzugsweise ist die Länge jedes Zeitfensters kleiner oder gleich einer Woche. Besonders bevorzugt wird als Zeitfenster ein Tag verwendet. Somit wird für jeden Tag, den der Nutzer das Hörgerätesystem verwendet, ein jeweiliges weiteres Zentralmaß ermittelt und somit einmalig der jeweilige Gesundheitszustand geschätzt. Mit anderen Worten wird einmal pro Tag bzw. für jeden Tag jeweils der Gesundheitszustand geschätzt. Somit ist einerseits ein Aufwand verringert. Andererseits ist dennoch ein sich schleichend verschlechternder Gesundheitszustand bestimmbar, weswegen der Nutzer vergleichsweise früh darauf hingewiesen werden kann.

Beispielsweise wird der Gesundheitszustand lediglich anhand des weiteren Zentralmaßes geschätzt. Zweckmäßigerweise wird eine statistische Eigenschaft des weiteren Zentralmaßes ermittelt, wie die Varianz oder eine Standardabweichung. Insbesondere diese beim Schätzen des Gesundheitszustands berücksichtigt. So wird beispielsweise bei einer vergleichsweise großen Unsicherheit, die in der statistischen Eigenschaft hinterlegt ist, wie einer großen Varianz, angenommen, dass keine oder lediglich eine geringe Veränderung des Gesundheitszustands vorliegt, sofern dieser auch anhand eines vorherigen bereits geschätzten Gesundheitszustands geschätzt wird. Zumindest jedoch wird eine vergleichsweise geringe Genauigkeit beim Schätzen des Gesundheitszustands angenommen. Falls dahingegen die Unsicherheit gering ist, wird eine vergleichsweise große Genauigkeit beim Schätzen des Gesundheitszustands angenommen.

Beispielsweise wird jeder ermittelte Temperaturwert zum Schätzen des Gesundheitszustands verwendet. Besonders bevorzugt jedoch wird überwacht, ob während des Ermittelns der Temperaturwerte der Nutzer eines der Hörgeräte zusätzlich berührt. Mit anderen Worten wird überwacht, ob zusätzlich zu der gewünschten Anlage des Hörgeräts an dem Nutzer eine weitere Berührung erfolgt, beispielsweise mit einer Hand oder einer sonstigen Extremität des Nutzers. Eine derartige Berührung erfolgt beispielsweise unbeabsichtigt, zum Anpassen der Position des jeweiligen Hörgeräts am Ohr oder zum Ändern einer Einstellung des jeweiligen Hörgeräts. Wenn der Nutzer das entsprechende Hörgerät berührt, wird dieser Temperaturwert beim Schätzen des Gesundheitszustands nicht berücksichtigt. Aufgrund der Berührung erfolgt nämlich zumindest eine geringfügige Änderung der Temperatur des jeweiligen Hörgeräts, die zu einer Verfälschung der jeweils ermittelten Abweichung führen kann. Somit wird ein derartiger Einfluss auf das Schätzen des Gesundheitszustands ausgeschlossen, weswegen eine Genauigkeit beim Schätzen erhöht ist. Insbesondere werden zudem für einen bestimmten Zeitraum nach dem Berühren des jeweiligen Hörgeräts durch den Nutzer die ermittelten Temperaturwerte nicht berücksichtigt, oder das Ermitteln wird ausgesetzt. Auf diese Weise wird der Gesundheitszustand somit über die Temperaturwerte lediglich Temperaturen berücksichtigt, die zu der in dem jeweiligen Ohr herrschenden Temperatur korrespondieren. Das Erfassen der Berührung erfolgt beispielsweise mittels Auswertens des mittels des etwaigen Mikrofons erstellten Audiosignals und/oder eines entsprechenden Sensors, wie beispielsweise eines Näherungssensors, oder eines Beschleunigungssensors. So wird beispielsweise bei einer vergleichsweise abrupten kurzzeitigen Änderung der Lage des Hörgeräts angenommen, dass eine entsprechende Berührung durch den Nutzer stattgefunden hat.

Beispielsweise wird der Gesundheitszustand lediglich anhand der Abweichung(-en) sowie beispielsweise anhand der etwaigen statistischen Eigenschaften geschätzt. Besonders bevorzugt jedoch werden noch weitere Parameter bei dem Schätzen des Gesundheitszustands berücksichtigt. Insbesondere werden hierbei Umgebungsparameter berücksichtigt, zweckmäßigerweise eine Umgebungstemperatur. Diese wird beispielsweise mittels eines entsprechenden Temperatursensors eines der Hörgeräte gemessen oder zweckmäßigerweise mittels eines Temperatursensors des Smartphones, sofern dieses ein Bestandteil des Hörgerätesystems ist. Geeigneterweise wird die Umgebungstemperatur vergleichsweise weit beabstandet von den Hörgeräten gemessen, sodass diese im Wesentlichen davon unabhängig ist. Mit anderen Worten wird die gemessene Umgebungstemperatur von dem Nutzer nicht oder zumindest von der Durchblutung dessen Gehirns nicht beeinflusst. Die Umgebungstemperatur wird vorzugsweise gleichzeitig zum Ermitteln der Temperaturwerte gemessen/erfasst. Falls die Umgebungstemperatur vergleichsweise gering, jedoch die Abweichung zwischen beiden Temperaturwerten vergleichsweise groß ist, befindet sich zum Beispiel eines der Ohren zumindest teilweise unter einer Mütze oder dergleichen. Anhand der Umgebungstemperatur ist dies ableitbar, sodass dies nicht zu einer Verfälschung beim Schätzen des Gesundheitszustands führt, sodass eine Genauigkeit erhöht ist.

Alternativ oder in Kombination hierzu wird bei Ermitteln der Temperaturwerte zusätzlich eine Nutzeraktivität erfasst, wofür zweckmäßigerweise ein geeigneter Sensor verwendet wird. Beispielsweise wird ein Herzfrequenzsensor oder Beschleunigungsmesser zur Charakterisierung einer sportlichen Aktivität verwendet. Die Nutzeraktivität wird bei der Ermittlung des Gesundheitszustands berücksichtigt. Insbesondere wird die Art der Nutzeraktivität und/oder die Intensität der Nutzeraktivität erfasst. Mit anderen Worten wird vorzugsweise der physiologische Zustand des Nutzers erfasst, der bei der Ermittlung des Gesundheitszustands berücksichtigt wird. So ist beispielsweise bei einer vergleichsweise schweren körperlichen Tätigkeit das Ausbilden einer vergleichsweise großen Abweichung möglich, ohne dass dies zu einem verschlechterten Gesundheitszustand korrespondiert.

Das Hörgerätesystem weist zwei Hörgeräte auf, die insbesondere als Hörhilfegeräte ausgestaltet sind. Geeigneterweise ist eines hiervon vorgesehen und eingerichtet, an oder in einem linken Ohr eines Nutzers und das andere an oder in einem rechten Ohr des Nutzers angeordnet zu werden. Jedes Hörgeräte weist einen Temperatursensor auf, wobei die beiden Temperatursensoren insbesondere zueinander gleichartig sind. Hierbei befinden sich im bestimmungsgemäßen Zustand jeder Temperatursensor beispielsweise im Gehörgang, vorzugsweise im Bereich eines Trommelfells des jeweiligen Ohrs, oder zumindest im Bereich / benachbart eines Temporallappens des Gehirns des Nutzers, zum Beispiel auf der Kopfoberfläche an einer Position, der dem Bereich eines Temporallappens entspricht. Vorzugsweise umfasst das Hörgerätesystem zudem ein tragbares Gerät, wie ein Smartphone, ein Tablett oder ein Wearable, mit dem die Hörgeräte signaltechnisch verbunden sind, vorzugsweise mittels einer Funkverbindung.

Das Hörgerätesystem ist gemäß einem Verfahren betrieben, bei dem mittels jedes Temperatursensors ein aktueller Temperaturwert ermittelt, vorzugsweise gleichzeitig. Eine Abweichung zwischen den beiden Temperaturwerte wird ermittelt, und anhand der Abweichung wird ein Gesundheitszustand des Nutzers des Hörgerätesystems geschätzt. Beispielsweise wird der geschätzte Gesundheitszustand mittels des etwaigen tragbaren Geräts ausgegeben oder dort zumindest abgespeichert. Vorzugsweise wird der Gesundheitszustand mittels des tragbaren Geräts geschätzt und/oder die Abweichung dort ermittelt. Hierbei werden bei dem Verfahren zunächst die Temperaturwerte wird von jedem Hörgerät zu dem tragbaren Gerät übermittelt, mittels dessen die entsprechende Weiterverarbeitung erfolgt. Somit sind benötigte Ressourcen bei den Hörgeräten verringert.

Die im Zusammenhang mit dem Verfahren beschriebenen Weiterbildungen und Vorteile sind sinngemäß auch auf das Hörgerätesystem zu übertragen und umgekehrt.

Nachfolgend wird ein Ausführungsbeispiel der Erfindung anhand einer Zeichnung näher erläutert. Darin zeigen:
- Fig. 1: schematisch einen Träger eines Hörgerätesystems, das zwei Hörgeräte und ein tragbares Gerät umfasst,
- Fig. 2: schematisch ausschnittsweise das Hörgerätesystem, und
- Fig. 3: ein Verfahren zum Betrieb des Hörgerätesystems.

Einander entsprechende Teile sind in allen Figuren mit den gleichen Bezugszeichen versehen.

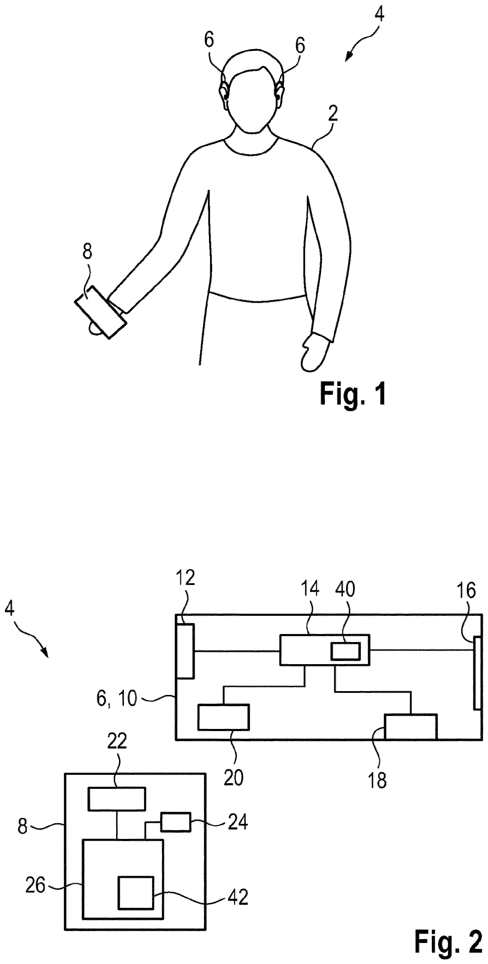

In Figur 1 ist schematisch vereinfacht ein Träger 2 eines Hörgerätesystems 4 dargestellt. Bei dem Träger 2 handelt es sich um einen Menschen (Person), der unter einem teilweisen Hörverlust leidet. Zur Abhilfe hiervon trägt der Träger 2 zwei Hörgeräte 6 des Hörgerätesystems 4. Dabei ist eines der Hörgeräte 6 einem linken Ohr und das andere Hörgerät 6 einem rechten Ohr des Nutzers 2 zugeordnet. Somit ist das Hörgerätesystem 4 binaural ausgestaltet. Das Hörgerätesystem 4 weist ferner ein tragbares Gerät 8 in Form eines Smartphones auf, das der Träger 2 ebenfalls mit sich führt.

Das Hörgerätesystem 4 ist in Figur 2 schematisch vereinfacht teilweise dargestellt. Hierbei ist lediglich eines der beiden Hörgeräte 6 gezeigt, die zueinander spiegelbildlich, aber ansonsten gleich, ausgestaltet sind. Das Hörgerät 6 ist in Form eines Hörhilfegeräts ausgebildet, das vorgesehen und eingerichtet ist, in den Gehörgang des jeweiligen Ohrs des Trägers (Nutzer, Benutzer, Hörgeräteträger) 2 eingeführt zu werden. Mit anderen Worten handelt es sich um ein "in-the-canal"-Hörhilfegerät (ITC- Hörhilfegerät). Das Hörgerät 6 umfasst ein Gehäuse 10, das aus einem Kunststoff gefertigt und auf die Form des jeweils zugeordneten Gehörgangs angepasst ist. Innerhalb des Gehäuses 10 ist ein Mikrofon 12 in Form eines elektromechanischen Schallwandlers angeordnet, das omnidirektional ausgestaltet sind. Das Mikrofon 12 ist mit einer Steuereinheit 14 signaltechnisch gekoppelt, die eine Signalverarbeitungseinheit mit einer nicht näher gezeigten Verstärkerschaltung und einem Signalprozessor umfasst. Die Steuereinheit 14 ist ferner mittels Schaltungselementen gebildet, wie zum Beispiel elektrischen und/oder elektronischen Bauteilen. Der Signalprozessor ist ein digitaler Signalprozessor (DSP) und über einen nicht näher dargestellten A/D-Wandler signaltechnisch mit dem Mikrofon 12 verbunden.

Mit der Steuereinheit 14 ist ein Hörer 16 gekoppelt, also signaltechnisch verbunden. Mittels des Hörers 16, der ein elektromechanischer Schallwandler ist, wird bei Betrieb ein mittels der Steuereinheit 14 bereitgestelltes Ausgabesignal in einen Ausgabeschall gewandelt, also in Schallwellen. Diese werden im bestimmungsgemäßen Gebrauch in den Gehörgang des jeweiligen Ohrs in Richtung des dort angeordneten Trommelfells eingeleitet.

In dem Gehäuse 10 ist ferner ein Temperatursensor 18 angeordnet. Der Temperatursensor 18 ist in einer Ausgestaltungsform ein temperaturabhängiger Widerstand, der in eine Öffnung des Gehäuses 10 eingebettet ist und im bestimmungsgemäßen Gebrauch an der Haut des Gehörgangs anliegt. Somit ist es möglich, mittels des Temperatursensors 18 die Temperatur der Haut des Nutzers 2 im Bereich des Gehörgangs zu messen. Bei einer alternativen Ausgestaltungsform ist der Temperatursensor 18 ein Infrarotsensor, der in Richtung des Trommelfells gerichtet ist. Somit ist es mittels des Temperatursensors 18 möglich, die Temperatur des Trommelfels berührungslos zu messen, die im Wesentlichen der Temperatur des der gleichen Seite zugeordneten Temporallappens des Gehirns des Nutzers 2 entspricht.

Zudem ist in dem Gehäuse 10 eine Kommunikationsvorrichtung 20 angeordnet, die dem Erstellen einer Funkverbindung mit dem tragbaren Gerät 8 dient. Die Kommunikationsvorrichtung 20 und somit auch die Funkverbindung 34 genügen hierbei einen Bluetooth-Standard. In dem Gehäuse 10 ist ferner einen nicht näher dargestellten Beschleunigungssensor angeordnet, mittels dessen eine Beschleunigung des Hörgeräts 6 erfasst werden kann, die beispielsweise aufgrund einer Bewegung des Gehäuses 10 bezüglich des Ohrs des Nutzers 2 oder aufgrund einer Bewegung des Nutzers 2 selbst hervorgerufen wird. Der Temperatursensor 18, der Beschleunigungssensor und die Kommunikationsvorrichtung 20 werden mittels der Steuereinheit 14 gesteuert und über diese bestromt. Mittels dieser erfolgt auch eine Versorgung des Mikrofons 12 sowie des Hörers 16 mit elektrischer Energie. Die Bestromung der Steuereinheit 14 erfolgt mittels einer in dem Gehäuse 10 angeordneten, nicht näher dargestellten Batterie.

Das als Smartphone ausgestaltete tragbare Gerät 8 weist eine weitere Kommunikationsvorrichtung 22 auf, die über die Funkverbindung bei Betrieb mit den Kommunikationsvorrichtungen 200 der beiden Hörgeräte 6 verbunden ist. Daher genügt die weitere Kommunikationsvorrichtung 22 dem Bluetooth-Standard. Das tragbare Gerät 8 umfasst einen weiteren Temperatursensor 24, mittels dessen eine Temperatur in der Umgebung des tragbaren Geräts 8 gemessen werden kann. Die weitere Kommunikationsvorrichtung 22 und der weitere Temperatursensor 20, 24 sind mit einer weiteren Steuereinheit 26 des tragbaren Geräts 8 signaltechnisch verbunden und werden mittels dieser betrieben.

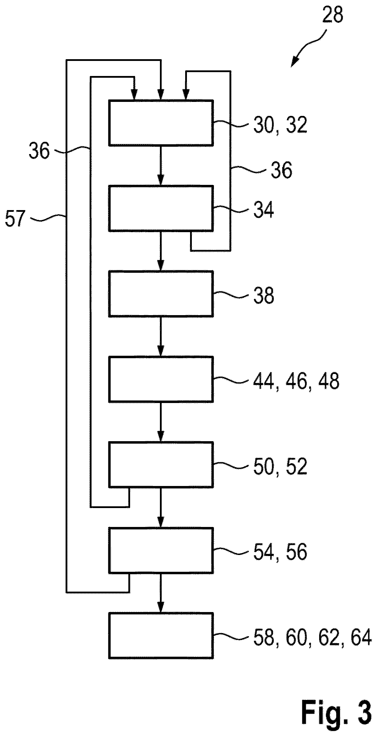

Die Steuereinheiten 14 sowie die weitere Steuereinheit 26 sind vorgesehen und eingerichtet, ein in Figur 3 dargestelltes Verfahren 28 zum Betrieb des Hörgerätesystems 4 durchzuführen. Mit anderen Worten ist das Hörgerätesystem 4 gemäß dem Verfahren 28 betrieben, und das Verfahren 28 wird mittels der jeweiligen Steuereinheiten 14, 26 zumindest teilweise durchgeführt. Hierbei wird in einem ersten Arbeitsschritt 30 mittels der beiden Temperatursensoren 18 ein jeweiliger aktueller Temperaturwert 32 ermittelt, der auch lediglich als Temperaturwert bezeichnet wird. Hierfür wird mittels jedes Temperatursensors 18 die Temperatur in dem Gehörgang gemessen, und der Messwert sowie der Zeitpunkt, zu dem das Messen der jeweiligen Temperatur erfolgte, werden in dem jeweiligen Temperaturwert 32 abgespeichert. Der jeweilige Zeitpunkt entspricht hierbei dem Zeitstempel des jeweiligen Temperaturwerts 32.

Mittels der beiden Hörgeräte 6 werden dabei die Temperaturen gleichzeitig mittels des jeweiligen Temperatursensors 18 gemessen, sodass die beiden Temperaturwerte 32 zeitgleich ermittelt werden. Zumindest ist ein Zeitversatz, der insbesondere anhand des Zeitstempels feststellbar ist, geringer als 1 Sekunde. Hierfür wurden vor Durchführung des Verfahrens 28 die beiden Hörgeräte 6 synchronisiert, sodass nicht näher dargestellte Zeitgeber der Hörgeräte 6 im Wesentlichen synchron betrieben werden.

In einem zweiten Arbeitsschritt 34 wird überprüft, ob bei Ermitteln der beiden Temperaturwerte 32 der Nutzer 2 eines der Hörgeräte 6 zusätzlich berührt hat, oder ob eine derartige Berührung innerhalb von 10 Sekunden vor Ermitteln der Temperaturwerte 32 stattgefunden hat. Hierfür werden die mittels des jeweiligen Mikrofons 12 erstellten Audiosignale auf ein Geräusch ausgewertet, das zu einer Berührung des Mikrofons 12 oder zumindest Gehäuses 10 mittels einer Extremität des Nutzers 2 korrespondiert. Auch werden die mittels des Beschleunigungssensors erfassten Signale hinsichtlich einer vergleichsweise abrupten und kurzen Bewegung des Gehäuses 10 analysiert. In diesem Fall wird ebenfalls davon ausgegangen, dass eine Berührung stattgefunden hat. Falls eine derartige Berührung stattgefunden hat, werden die ermittelten Temperaturwerte 32 verworfen, und nach einem zeitlichen Abstand 36 wird erneut der erste Arbeitsschritt 30 durchgeführt. Der zeitliche Abstand 36 beträgt dabei stets 1 Stunde.

Zusammenfassend wird somit überwacht, ob während des Ermittelns der Temperaturwerte 32 der Nutzer 2 eines der Hörgeräte 6 zusätzlich berührt, also ob auch zusätzlich zur Berührung des jeweiligen Ohrs noch eine weitere Berührung des Hörgeräts 6 durch den Nutzer 2 erfolgt. In diesem Fall werden die ermittelten Temperaturwerte 32 nicht berücksichtigt. Anderenfalls wird ein dritter Arbeitsschritt 38 durchgeführt. In diesem wird von jedem Hörgerät 6 der jeweils ermittelte aktuelle Temperaturwert 32 zunächst in einem Speicher 40 der jeweiligen Steuereinheit 40 abgespeichert. Zudem wird der aktuelle Temperaturwert 32 von der Steuereinheit 14 mittels der Kommunikationsvorrichtung 20 zu dem tragbaren Gerät 8 übertragen und dort mittels der weitere Kommunikationsvorrichtung 22 empfangen. Die empfangen Temperaturwerte 32 werden in einem weiteren Speicher 42 der weiteren Steuereinheit 26 hinterlegt. Falls bei der Übermittlung der Temperaturwerte 32 ein Fehler auftreten sollte, ist ein erneuter Austausch möglich, da diese in dem jeweiligen Speicher 40, 42 hinterlegt sind.

In einem vierten Arbeitsschritt 44 wird mittels des weiteren Temperatursensors 24 eine Umgebungstemperatur 46 erfasst. Auch wird in dem vierten Arbeitsschritt 44 eine Nutzeraktivität 48 erfasst. Hierfür werden von dem tragbaren Gerät 8, nämlich der weiteren Steuereinheit 26, die Beschleunigungssensoren der beiden Hörgeräte 6 abgefragt und ein nicht näher dargestellte Beschleunigungssensor des tragbaren Geräts 8 ausgelesen. Anhand der sich in den Messignalen enthaltenen Muster wird eine Art und eine Intensität der bei Ermitteln der Temperaturwerte 32 durchgeführten Nutzeraktivität 48 bestimmt. Zusammenfassend wird somit bei Ermitteln der Temperaturwerte 32 zusätzlich die Umgebungstemperatur 46 erfasst und die dann vorherrschende Nutzeraktivität 48. Diese wird mit dem jeweiligen Zeitstempel versehen in dem weiteren Speicher 42 abgespeichert.

In einem sich anschließenden fünften Arbeitsschritt 50 wird eine Abweichung 52 zwischen den Temperaturwerten 32 ermittelt. Hierfür wird von dem Temperaturwert 32, der mittels des dem linken Ohr des Nutzers 2 zugeordneten Hörgerät 6 ermittelt wurde, der Temperaturwert 32 abgezogen, der mittels des dem rechten Ohr des Nutzers 2 zugeordneten Hörgerät 6 ermittelt wurde. Die ermittelte Abweichung 52 wird mit dem jeweiligen Zeitstempel versehen und ebenfalls in dem weiteren Speicher 42 hinterlegt. Nach Ablauf des zeitlichen Abstand 36, also nach einer Stunde, wird wiederum der erste Arbeitsschritt 32 durchgeführt.

Nach zehnmaligem Durchführen des ersten Arbeitsschritt 32, also nach 10 Stunden, wird ein sechster Arbeitsschritt 54 durchgeführt. In diesem wird ein Zentralmaß 56 der in den 10 vorhergehenden Stunden ermittelten Abweichungen 52 ermittelt. Das Zentralmaß 56 entspricht hierbei dem Median der Abweichungen 52. Nachfolgend wird wiederum der erste Arbeitsschritt 32 durchgeführt.

Nach einem Zeitfenster 57, das eine Woche beträgt, wird ein siebter Arbeitsschritt 58 durchgeführt. In diesem wird ein weiteres Zentralmaß 60 der bis zu diesem Zeitpunkt ermittelten Zentralmaße 56 erstellt. Als weiteres Zentralmaß 60 wird hierbei der arithmetische Mittelwert der Zentralmaße 56 verwendet. Zudem wird eine statistische Eigenschaft 62, nämlich die Varianz des weiteren Zentralmaßes 60 ermittelt.

Anhand des weiteren Zentralmaßes 60 wird ein Gesundheitszustand 64 des Nutzers geschätzt. Da die Zentralmaße 56 verwendet werden, wird jeder Temperaturwert 32 somit mehrmals gemessen und jeweils die Abweichung 52 ermittelt, und anhand der Abweichungen 52 wird der Gesundheitszustand 64 des Nutzers 2 geschätzt. Dabei wird für die mehreren aufeinanderfolgenden Zeitfenster 57 jeweils ein entsprechendes Zentralmaß 56 erstellt, wobei der Gesundheitszustand 64 anhand des weiteren Zentralmaßes 60 geschätzt wird, das anhand der Zentralmaße 56 erstellt wurde.

Falls nämlich die dem linken Ohr des Nutzers 2 zugeordneten Temperaturwerte 32 signifikant geringer als die dem rechten Ohr des Nutzers 2 zugeordneten Temperaturwerte 32 sind, was anhand des weiteren Zentralmaßes 60 bestimmt ist, ist die Wahrscheinlichkeit, dass die linke Gehirnhälfte des Nutzers 2 schlechter durchblutet ist erhöht. In diesem Fall ist das weitere Zentralmaß 60 negativ. Da die Zentralmaße 56 verwendet werden, wird jeder Temperaturwert 32 somit mehrmals gemessen und jeweils die Abweichung 52 ermittelt, und anhand der Abweichungen 52 wird der Gesundheitszustand 64 des Nutzers 2 geschätzt.

Eine verringerte Durchblutung der linken Gehirnhälfte korrespondiert zu einer verringerten neuronalen Aktivität der linken Gehirnhälfte. Dies ist zumindest meist auch der Fall, wenn der Nutzer 2 unter einer Depression leidet oder diese beginnt, sowie zum Beispiel bei einem schizoiden Verhalten oder einem anderen sozialen Rückzug. Dabei wird bei dem Schätzen des Gesundheitszustands 64 auch die statistische Eigenschaft 62, nämlich die Varianz berücksichtigt. Falls diese vergleichsweise groß ist, wird die Wahrscheinlichkeit, dass eine Verschlechterung des Gesundheitszustands 64 vorliegt, also insbesondere das eine geschätzte Depression oder dergleichen vorliegt, verringert. Falls dahingegen eine kleine Varianz vorhanden ist, ist die Wahrscheinlichkeit für das Vorliegen erhöht.

Bei dem Schätzen des Gesundheitszustands wird dabei zudem die jeweilige Umgebungstemperatur 46 berücksichtigt. So wird bei einer vergleichsweise niedrigen Umgebungstemperatur 46 und vergleichsweise großen Abweichungen 52 eine verringerte Wahrscheinlichkeit, dass eine Erkrankung, also eine Verschlechterung des Gesundheitszustands 60 vorliegt, angenommen. Es ist nämlich möglich, dass bei niedrigen Umgebungstemperaturen 46 der Nutzer 2 eine Mütze oder dergleichen verwendet, wobei diese beispielsweise lediglich über eines der beiden Ohren gezogen ist.

Ferner wird beim Ermitteln beim Schätzen des Gesundheitszustands 64 auch die ermittelte Nutzeraktivität 48 berücksichtigt. Bei dem Vorliegen einer schweren körperlichen Tätigkeit können nämlich vergleichsweise große Abweichungen 52 vorliegen, ohne dass eine Verschlechterung des Gesundheitszustands 64 vorhanden ist. Falls somit eine schwere körperliche Tätigkeit als Nutzeraktivität 48 vorliegt, erfolgt eine geringere Gewichtung der dann ermittelten Abweichungen 52.

Falls ein verschlechterter Gesundheitszustand 64 des Nutzers 2 geschätzt wird, wird mittels des tragbaren Geräts 8 eine entsprechende Meldung ausgegeben, sodass der Nutzer 2 eine Medizinische Fachperson aufsuchen kann. Dieser kann dann eine Untersuchung des Nutzers 2 vornehmen und eine entsprechende Diagnose stellen. Auf diese Weise ist es für die medizinische Fachperson erleichtert, eine beginnende Depression zu diagnostizieren, zumal ein Hinweis auf das mögliche Vorhandensein vorliegt.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit dem Ausführungsbeispiel beschriebene Einzelmerkmale auch auf andere Weise miteinander kombinierbar, ohne den Gegenstand der Erfindung zu verlassen.

### Bezugszeichenliste

- 2: Träger
- 4: Hörgerätesystem
- 6: Hörgerät
- 8: tragbares Gerät
- 10: Gehäuse
- 12: Mikrofon
- 14: Steuereinheit
- 16: Hörer
- 18: Temperatursensor
- 20: Kommunikationsvorrichtung
- 22: weitere Kommunikationsvorrichtung
- 24: weiterer Temperatursensor
- 26: weiterer Steuereinheit
- 28: Verfahren
- 30: erster Arbeitsschritt
- 32: aktueller Temperaturwert
- 34: zweiter Arbeitsschritt
- 36: zeitlicher Abstand
- 38: dritter Arbeitsschritt
- 40: Speicher
- 42: weiterer Speicher
- 44: vierter Arbeitsschritt
- 46: Umgebungstemperatur
- 48: Nutzeraktivität
- 50: fünfter Arbeitsschritt
- 52: Abweichung
- 54: sechster Arbeitsschritt
- 56: Zentralmaß
- 57: Zeitfenster
- 58: siebter Arbeitsschritt
- 60: weiteres Zentralmaß
- 62: statistische Eigenschaft
- 64: Gesundheitszustand

## Patentansprüche

1. Verfahren (28) zum Betrieb eines Hörgerätesystems (4), das zwei Hörgeräte (6), insbesondere Hörhilfegeräte, mit jeweils einem Temperatursensor (18) aufweist, bei welchem
- mittels jedes Temperatursensors (18) ein aktueller Temperaturwert (32) ermittelt wird,
- eine Abweichung (52) zwischen den beiden Temperaturwerten (32) ermittelt wird,
- anhand der Abweichung (52) ein Gesundheitszustand (64) eines Nutzers (2) des Hörgerätesystems (4) geschätzt wird.

2. Verfahren (28) nach Anspruch 1,
**dadurch gekennzeichnet,**
**dass** die beiden Temperaturwerte (32) zeitgleich ermittelt werden.

3. Verfahren (28) nach Anspruch 1 oder 2,
**dadurch gekennzeichnet,**
**dass** jeder Temperaturwert (32) mehrmals gemessen und jeweils die Abweichung (52) ermittelt wird, wobei der Gesundheitszustand (64) anhand eines Zentralmaßes (56) der Abweichungen geschätzt wird.

4. Verfahren (28) nach Anspruch 3,
**dadurch gekennzeichnet,**
**dass** für mehrere aufeinanderfolgende Zeitfenster (57) jeweils ein derartiges Zentralmaß (56) erstellt wird, wobei der Gesundheitszustand (64) anhand eines weiteren Zentralmaßes (60) der Zentralmaße (56) geschätzt wird.

5. Verfahren (28) nach Anspruch 4,
**dadurch gekennzeichnet,**
**dass** eine statistische Eigenschaft (62) des weiteren Zentralmaßes (60) ermittelt wird, die bei dem Schätzen des Gesundheitszustands (64) berücksichtigt wird.

6. Verfahren (28) nach einem der Ansprüche 1 bis 5,
**dadurch gekennzeichnet,**
**dass** überwacht wird, ob während des Ermittelns der Temperaturwerte (32) der Nutzer (2) eines der Hörgeräte (6) zusätzlich berührt, wobei die dann ermittelten Temperaturwerte (32) nicht berücksichtigt werden.

7. Verfahren (28) nach einem der Ansprüche 1 bis 6,
**dadurch gekennzeichnet,**
**dass** bei Ermitteln der Temperaturwerte (32) zusätzlich eine Umgebungstemperatur (46) erfasst wird, die bei dem Schätzen des Gesundheitszustands (64) berücksichtigt wird.

8. Verfahren (28) nach einem der Ansprüche 1 bis 7,
**dadurch gekennzeichnet,**
**dass** bei Ermitteln der Temperaturwerte (32) zusätzlich eine Nutzeraktivität (48) erfasst wird, die bei der Ermittlung des Gesundheitszustands (64) berücksichtigt wird.

9. Hörgerätesystem (4), das zwei Hörgeräte (4), insbesondere Hörhilfegeräte, mit jeweils einem Temperatursensor (18) aufweist, und das gemäß einem Verfahren (28) nach einem der Ansprüche 1 bis 8 betrieben ist.
